**Europäisches Patentamt**

**European Patent Office**

**Office européen des brevets**

(19)

(11) **EP 1 417 978 A1**

(12) **EUROPEAN PATENT APPLICATION**
published in accordance with Art. 158(3) EPC

(43) Date of publication:
**12.05.2004 Bulletin 2004/20**

(51) Int Cl.⁷: **A61L 9/015**, A61L 9/00,
A61L 9/014

(21) Application number: **01272901.8**

(22) Date of filing: **27.12.2001**

(86) International application number:
**PCT/JP2001/011558**

(87) International publication number:
**WO 2002/053196 (11.07.2002 Gazette 2002/28)**

(84) Designated Contracting States:
**AT BE CH CY DE FR GB IT LI**

(30) Priority: **28.12.2000 JP 2000402908
25.09.2001 JP 2001292441**

(71) Applicant: **TOSHIBA LIGHTING & TECHNOLOGY
CORPORATION
Shinagawa-ku, Tokyo 140-8640 (JP)**

(72) Inventors:
• **SAITOU, Akiko
Yokohama-Shi, Kanagawa 226-0021 (JP)**
• **MATSUDA, Ryoutarou
Yokosuka-Shi, Kanagawa 239-0808 (JP)**
• **ISHIZAKI, Ariyoshi
Yokohama-Shi, Kanagawa 240-0024 (JP)**
• **OTSUKA, Kazunari
Yokohama-Shi, Kanagawa 223-0053 (JP)**
• **OISHI, Takanobu
Saitama-Shi, Saitama 336-0037 (JP)**

(74) Representative: **Kramer - Barske - Schmidtchen
European Patent Attorneys
Patenta
Radeckestrasse 43
81245 München (DE)**

(54) **DEODORIZING DEVICE**

(57)    A deodorization apparatus comprises: a case body 2a formed therein with an air-flow passage 2i through which air flows; first and second lamp units 8, 10 disposed in an air-flow passage in the case body and adapted to generate ozone; first, second and third photo-catalyst filters 7, 9, 11 disposed in the air-flow passage in the case body and provided with photo-catalyst filters; and an impregnated carbon disposed on the downstream side of at least one of the first and second lamp units 8, 10 and the photo-catalyst filters and formed by impregnating an activated carbon adsorbing an odor component with at least one component of iron oxide, potassium hydroxide, nickel nitrate. According to this structure, the deodorization effect at the one passing of the contaminated air, in the air-flow passage, containing odor component is improved, the usable life time can be elongated, and the maintenance of the impregnated carbon can be simplified.

FIG. 1

1   DEODORIZATION APPARATUS
2   CASE BODY
2a  CASE BODY PORTION
2e  OPENED UPPER END OF CASE BODY PORTION
2d  UPPER LID OF CASE BODY PORTION
2d  PACKING OF UPPER LID
2f, 2g SIDE WALL
2h  INNER BOTTOM SURFACE
2i  AIR-FLOW PASSAGE
3   INTAKE PIPE
5   EXHAUST FAN
6   WATER REMOVAL AIR-FILTER
7   FIRST PHOTO-CATALYST FILTER
8   FIRST LAMP UNIT
9   SECOND PHOTO-CATALYST FILTER
10  SECOND LAMP UNIT
11  THIRD PHOTO-CATALYST FILTER
12  ADSORBENT UNIT
13  ULTRAVIOLET LAMP
15  ELECTRICAL PART BOX
16a,16b A PAIR OF GUIDE RAILS
17  LERF SPRING

## Description

Technical Field

The present invention relates to a deodorization apparatus for removing odor component of an odor gas.

Background Art

In a conventional art, one example of such deodorization apparatus is disclosed in Japanese Patent Laid-open Publication 2000-316961. This deodorization apparatus includes a photo-catalyst layer disposed in a contaminated air passage and adapted to adsorb organic compound and nitride and to oxidize and decompose them, and a sulfur containing odor deodorizing layer disposed downstream side of the photo-catalyst layer.

Thus, according to such deodorization apparatus, an odor component mainly consisting of acetaldehyde gas is removed and decomposed by a photo-catalyst in the photo-catalyst layer, and the sulfur containing odor component is deodorized by the sulfur containing odor deodorizing layer disposed downstream side the photo-catalyst layer, thus achieving deodorizing function against odor gas containing various odor components.

However, in such conventional deodorization apparatus, since acetaldehyde or like is removed and decomposed by a photo-catalyst in the photo-catalyst layer, and on the other hand, the sulfur containing odor component is deodorized in the sulfur containing odor deodorizing layer and odor components deodorized by the photo-catalyst layer and the sulfur containing deodorizing layer are different from each other, so that the odor component containing acetaldehyde or like and the sulfur containing odor component are deodorized only one time, respectively, during one passing of the contaminated air containing these odor components through the photo-catalyst layer and the sulfur containing odor deodorizing layer. Therefore, it has been said that the respective odor components cannot be sufficiently deodorized and removed, thus providing a problem.

Japanese Patent Laid-open Publication No. HEI 9-206558 discloses another one example of conventional deodorization apparatus which is provided with an ozonation material disposed downstream side of a deodorization unit composed in combination of photo-catalyst and ozone. However, since this unit merely utilizes activated carbon as ozonation material, deodorization effect to the sulfur containing odor component is not sufficient.

The present invention was conceived in consideration of the above circumstances, and its object is to provide an deodorization apparatus capable of improving deodorization effect at a time when contaminated air containing odor components is passed one time through an air-flow passage, elongating a usable life time of adsorbent and making simple maintenance of the apparatus.

Disclosure of The Invention

In order to achieve the above object, the present invention provides a deodorization apparatus which is characterized by comprising:

a case body formed therein with an air-flow passage through which air flows;
an ozone generator, for generating ozone, disposed in an air-flow passage in the case body;
a photo-catalyst filter unit disposed in the air-flow passage in the case body and provided with a photo-catalyst filter; and
an impregnated carbon disposed on a downstream side of at least one of the ozone generator and the photo-catalyst filter and formed by impregnating an activated carbon absorbing an odor component with at least one component of iron oxide, potassium hydroxide, nickel nitrate.

The deodorization apparatus of this invention is effective for the deodorization of sulfur (or sulfur containing) odor. Further, in the following description, sulfur odor gas means an odor gas including sulfur (S) in its chemical formula. Furthermore, the photo-catalyst filter unit may be composed of a photo-catalyst filter and a light source for energizing the filter, which is mounted thereto as a unit, or the light source may be disposed independently. Still furthermore, the sun light may be utilized as such light source for the photo-catalyst filter.

According to the present invention, when the contaminated air containing odor gas passes through an air passage formed in the body case, the odor component is oxidized and decomposed by the ozone generated by the ozone generator to be thereby deodorized, then further oxidized and decomposed by the photo-catalyst filters and further deodorized through the adsorption by means of the impregnated carbon. Accordingly, through one-pass flowing of the contaminated air in the air-flow passage in the case body, the odor component in the contaminated air is deodorized three times by the ozone generator, the photo-catalyst filters and the impregnated carbon, so that hydrogen sulfide $H_2S$, methylmercaptan $CH_3SH$, ethylmercaptan $C_2H_5SH$, methyl sulfide $H_3SCH_3$, and methyl disulfide $CH_3SSCH_3$ can be deodorized with high efficiency.

Furthermore, according to the present invention, additional remarkable effects, such as mentioned below, will be achieved more than mere the deodorization effect of each of ozone generator, the photo-catalyst filter and the impregnated carbon.

In the case where the impregnated carbon is formed by impregnating the activated carbon with potassium hydroxide (KOH), since the potassium hydroxide has an alkaline property, sulfur odor having an acidic property will be easily captured as shown by the following equation (1).

$$H_2S + KOH \rightarrow K\text{-}HS + H_2O \qquad (1)$$

In the case of only the activated carbon, in an ozone atmosphere, this activated carbon (C) will be easily oxidized by the ozone ($O_3$) as shown by the following equation (2). This activated carbon is likely oxidized and deteriorated.

$$2O_3 + C \rightarrow 2O_2 + CO_2 \qquad (2)$$

However, in the present embodiment, potassium hydroxide exists on the activated carbon surface, so that the deterioration due to the oxidization of the activated carbon by the ozone can be substantially prevented or reduced by this potassium hydroxide, thus elongating a usable life time of the activated carbon.

Moreover, in the case where the sulfur odor component consists of hydrogen sulfide, the ozone and the hydrogen sulfide react, and sulfur dioxide gas is generated as shown by the following equation (3) and sulfur is also generated as shown by the following equation (4).

$$H_2S + O_3 \rightarrow H_2O + SO_2 \qquad (3)$$

$$H_2S + O_3 \rightarrow S + H_2O + O_2 \qquad (4)$$

However, the sulfur dioxide gas and the sulfur are subjected, in the body case, to the deodorization treatment by the ozone, the photo-catalyst or the impregnated carbon, so that the external discharge of the sulfur odor component can be prevented or substantially reduced.

Furthermore, in a case where the impregnated carbon has a composition formed by impregnated activated carbon with iron oxide or nickel nitrate, sulfur odor component can be also effectively deodorized. That is, the iron oxide ($Fe_2O_3$) decomposes the hydrogen sulfide by desulfurizing function represented by the following equation (5).

$$Fe_2O_3 + 3H_2S \rightarrow Fe_2S_3 + 3H_2O \qquad (5)$$

Further, the nickel nitrate can promote the adsorption of the sulfur odor component to the activated carbon through its catalyst reaction, and also can decompose the ozone into oxygen, so that the discharging of the odor component outside the case body can be prevented or substantially reduced.

Furthermore, since the impregnated carbon is disposed on the downstream side of the ozone generator and the photo-catalyst filter, when the contaminated air passes through the impregnated carbon, the odor component has been deodorized twice by the ozone generator and the photo-catalyst filter, so that the odor density has been reduced on the upstream side of the impregnated carbon. For this reason, the quantity of the odor component to be adsorbed by the impregnated carbon can be reduced, so that the usable life time of the impregnated carbon can be elongated, and the requirement for the impregnated carbon to be exchanged will be reduced, contributing simplification of maintenance.

Further, in the deodorization apparatus of the characters mentioned above, it is desired that the ozone generator is a light source radiating a light having wavelengths of 185 nm and 254 nm.

According to this feature, the ozone generator is a light source irradiating ultraviolet ray having wavelength of 185 nm and 254 nm, this light source is cheep and light weight in comparison with a corona discharge device or like, and maintenance can be easily done, so that the cost as the deodorization apparatus can be reduced and the structure thereof can be made compact in light weight, and in addition, the maintenance and the exchanging of the light source can be easily done.

That is, in a known ozone generator for generating ozone by the corona discharge, a by-product such as nitrogen oxide is produced at the corona discharging time, so that it is necessary to provide an additional means for removing such by-product, which results in enlargement of the apparatus and cost increasing. On the contrary, according to the use of the ultraviolet lightening source, no by-product is formed, so that the apparatus can be made compact and production cost can be reduced.

Furthermore, in this deodorization apparatus, it is desired that the photo-catalyst filter is formed by fixing a photo-catalyst to a glass wool by means of fluororesin.

According to this feature, since the photo-catalyst filter is constructed by fixing a photo-catalyst to a glass wool by means of fluororesin, it can be washed with water. Therefore, by washing catalyst poison, which is formed by a photo-catalyst of the photo-catalyst filter to which dust or like adheres, the photo-catalyst filter can be easily reproduced at any time.

Furthermore, in the deodorization apparatus, it is also desired that the light sources for generating the ozone are disposed to both side portions of the photo-catalyst filter in the air-flow direction.

According to this structure, since the ozone generating light sources are disposed to both the sides of the photo-catalyst filter in the air-flow direction, odor gas can be deodorized twice at the upstream and downstream sides of the photo-catalyst filters. In addition, since the photo-catalyst film of the photo-catalyst filter can be excited and activated by the ultraviolet rays or ozone irradiated from the ozone generating light source, the deodorization effect by the photo-catalyst film can be further improved.

Furthermore, in the deodorization apparatus, it is desired that the impregnated carbon is accommodated in a container, through which the air passes, and the impregnated carbon container and the photo-catalyst filter are formed into a unit to be detachable to the case body.

According to this feature, the impregnated carbon unit and the photo-catalyst filter unit are detachably mounted to the case body, so that the impregnated carbon and the photo-catalyst filter can be easily and quickly exchanged to thereby simplify the maintenance work.

Furthermore, for the deodorization apparatus, it is also desired to include a support member for detachably supporting the impregnated carbon unit and the photo-catalyst filter in the case body and an elastic member for elastically supporting the impregnated carbon unit and the photo-catalyst filter in the support member.

According to this feature, the impregnated carbon unit and the photo-catalyst filter in the support member are elastically supported by the elastic member in the case body, so that looseness of the impregnated carbon unit and the photo-catalyst filter unit in the support member in the case body by the air-flow therein can be prevented, and the formation of space or gap between the inner surface of the support member and the impregnated carbon unit and the photo-catalyst filter unit can be also prevented, so that the quantity of the air-flow through the impregnated carbon unit and the photo-catalyst filter unit can be reduced. That is, substantially all the air-flow quantity can pass through the impregnated carbon unit and the photo-catalyst filter unit.

Furthermore, in the deodorization apparatus, it is desired that the photo-catalyst filter has a bent surface of which air-flow surface is bent.

According to this feature, since the air-flow surface of the photo-catalyst filter is bent, the air-flow surface area of the photo-catalyst filter, i.e., the surface area of the photo-catalyst film can be enlarged, and accordingly, the deodorization function of the photo-catalyst filter can be improved.

Furthermore, in the deodorization apparatus, it is desired that an exhaust means for externally exhausting air in the case body is disposed downstream side of the impregnated carbon.

According to this structure, since the exhaust means is disposed on the downstream side of the impregnated carbon, the air-flowing efficiency inside the case body can be improved, and in addition thereto, the air flows through the ozone generator, the photo-catalyst filter and the impregnated carbon which have the deodorization function can be realized, thus improving the deodorization efficiency.

Furthermore, for the deodorization apparatus, it is desired to further include a safety device disposed in the case body for blocking or stopping an operation of the ozone generator at a time of detecting that the ozone generated by the ozone generator leaks outside the case body.

Further, the term "leak" of the ozone outside the case body herein means a case that the leak amount of the ozone, leaking outside the case body during the operation of the deodorization apparatus, exceeds an allowable value. This allowable value is a value prescribed, by Japanese Industrial Sanitary Association (NIPPON SANGYOU EISEI GAKKAI), to be an average allowable ozone density of 0.1 ppm in a sealed state as a reference value in a working environment (work: 8 hours/day and 40 hours/week). As the ozone generator, there may be adopted either one of metal fine wire seal type, metal oxide powder seal type, diffusion-drift type, nitrogen discharge light silent discharge superimposing type, rotating electrode type, cryogenic operative glow discharge type, double-discharge type, corona discharge type, and electrolytic method type ozone generators.

In the case where the safety device detects a state that the ozone generated by the ozone generator leaks outside the case body, a state that the open/close lid of the case body is maintained to be opened, and a state that, because the impregnated carbon unit is not disposed to a predetermined portion in the case body for the exchanging of the impregnated carbon or like, the ozone is not adsorbed by the impregnated carbon and hence leaks outside the case body, the operation of the ozone generator can be forcibly stopped prior to the operation thereof by this safety device, or during the operation thereof, the operation is stopped. For this reason, since the generation of the ozone is blocked or stopped, the amount of the ozone leaking outside the case body can be prevented or reduced from exceeding the allowable value. As a result, the safeness to the operator operating this deodorization apparatus can be improved.

Furthermore, in this deodorization apparatus, it is desired that when an air-flow rate in the case body is 10 to 18 $m^3$/hr, an impregnated carbon having a grain diameter of 4 to 6 mm fills a unit case having a thickness of 70 to 130 mm in the air-flow direction, the unit case being detachably mounted to the case body.

Further, it is to be herein noted that the term "air-flow quantity or amount" is obtainable from the air flow velocity and the sectional area of the air-flow passage.

According to this feature, since, in the impregnated carbon unit, the impregnated carbon having a grain diameter of 4 to 6 mm fills the unit case having a thickness in the air flow direction of 70 to 130 mm at the time of the air-flow amount in the air-flow passage of the case body of 10 to 18 $m^3$/hr, the deodorization amount of the contaminated air can be increased while reducing the pressure loss at the air-flow time of the impregnated carbon unit.

That is, in the case of more than 130 mm of the thickness of the impregnated carbon unit in the air-flow direction, the air-flow resistance increases, the air-flow pressure loss of the activated carbon unit, and air-flowing ability of the air-flowing device is lowered, so that the contaminated air flow amount in the air-flow passage is reduced and the contaminated air may leak on the side of the air-flow inlet of the case body, thus being inconvenient.

On the other hand, in the case of less than 70 mm of the thickness of the impregnated carbon unit in the air-flow direction, the pressure loss can be reduced, but since the filling amount of the impregnated carbon is reduced, the contaminated air leaking outside the case body may increase, thus being also inconvenient.

Accordingly, as mentioned above, by setting the thickness of the impregnated carbon unit in the air-flow direction to 70 to 130 mm, the quantity of deodorization of the contaminated air can be increased while reducing the pressure loss in the air-flow time of the impregnated carbon unit.

Furthermore, in this deodorization apparatus, it is desired for the impregnated carbon unit to have a structure adjustable in its thickness in the air-flow direction.

According to this structure, since the thickness of the impregnated carbon unit is adjustable in the air-flow direction, by suitably adjusting the thickness thereof in the air-flow direction in accordance with the change of the quantity of odor to be treated of the contaminated air, the filling amount of the impregnated carbon can be easily and quickly adjusted.

Furthermore, in this deodorization apparatus, the case body is provided with an open/close lid for the case body and the safety device, mentioned above, comprises a first detection means for blocking or stopping the operation of the ozone generator upon detection of an opened state of the open/close lid.

According to this structure, in the case where the open/close lid of the case body is opened during the operation of the deodorization apparatus, the opened state is detected by, for example, the first detection means of the safety device such as limit switch and, in addition, the driving power source of the ozone generator is made off by this first detection means to thereby forcibly block or stop the operation thereof.

According to this structure, since the ozone generation in the case body is blocked or stopped, the leaking of the ozone outside the case body can be prevented or significantly reduced.

Still furthermore, in this deodorization apparatus, it is desired for the safety device to further include a second detection means for blocking or stopping the operation of the ozone generator upon detection of a state that the impregnated carbon unit is mounted to a portion other than a predetermined position in the case body.

According to this structure, since the generation of the ozone in the case body can be blocked or stopped, the leak amount of the ozone leaking outside the case body can be prevented or reduced from exceeding an allowable value.

Still furthermore, in this deodorization apparatus, it is desired for the first and second detection means to be disposed downstream side of the impregnated carbon in the air-flow direction.

According to this structure, since the first and second detection means are disposed downstream side the impregnated carbon unit, for adsorbing the odor component in the contaminated air and ozone, in the air-flow direction, these first and second detection means can be prevented from being directly exposed to the odor component and ozone.

Because of this reason, the first and second detection means can be prevented or reduced from being oxidized and deteriorated by the ozone of acidic gas in the odor component.

Brief Description of The Drawings

Fig. 1 is a plan view of a deodorization apparatus according to a first embodiment of the present invention in which an upper lid is removed.

Fig. 2 is a sectional view taken along the line II-II in Fig. 1 in a state that the upper lid of the deodorization apparatus of Fig. 1 is mounted.

Fig. 3 is a right side view, partially in section, of the deodorization apparatus shown in Figs. 1 and 2.

Fig. 4 shows a graph representing removal ratio of acetaldehyde by means of ozone in the deodorization apparatus of Fig. 1.

Fig. 5 is a graph showing deodorization effect of hydrogen sulfide of the deodorization apparatus shown in Fig. 1.

Fig. 6 is a plan view of a deodorization apparatus according to a second embodiment of the present invention in which an upper lid is removed.

Fig. 7 is a sectional view taken along the line VII-VII in Fig. 6 in a state that the upper lid of the deodorization apparatus of Fig. 6 is mounted.

Fig. 8 is a right side view, partially in section, of the deodorization apparatus shown in Figs. 6 and 7.

Fig. 9 is a table showing a relationship between thickness of an impregnated carbon unit of the deodorization apparatus, shown in Fig. 6, in an air-flow direction and an air-flow rate.

Fig. 10 is a graph showing the relationship between the thickness of an impregnated carbon unit of the deodorization apparatus, shown in Fig. 6, in an air-flow direction and the air-flow rate.

Fig. 11 is an illustration of an essential portion for explanation of a function of a limit switch for detecting the upper lid shown in Fig. 6.

Fig. 12 is an illustration of an essential portion for explanation of a function of a limit switch for detecting the impregnated carbon shown in Fig. 6.

Best Mode for Embodying The Invention

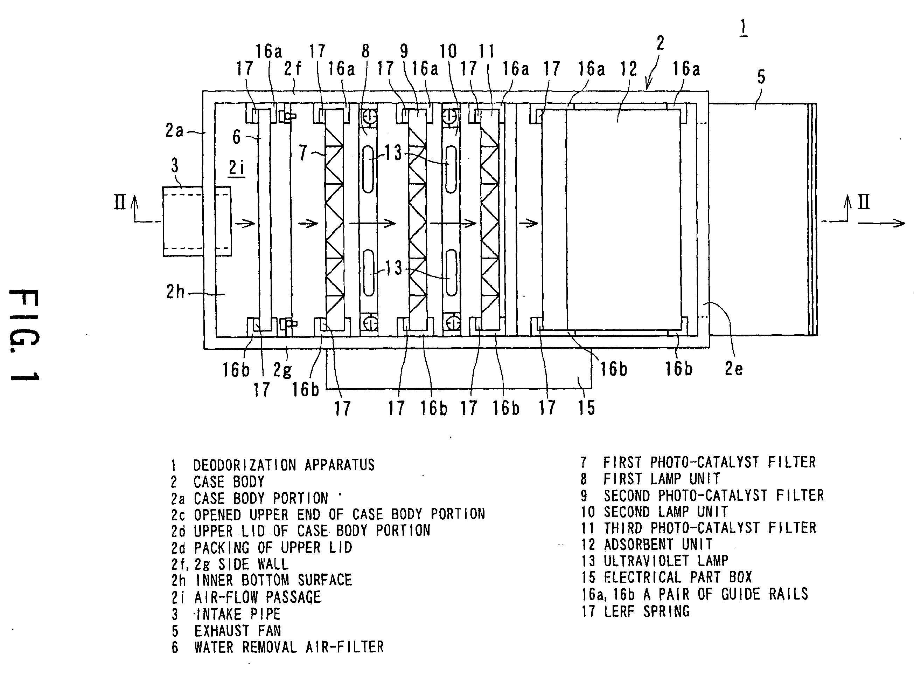

Hereunder, preferred embodiments of the present invention will be described with reference to Figs. 1 to 12, in which like reference numerals are added to the same or equivalent portions.

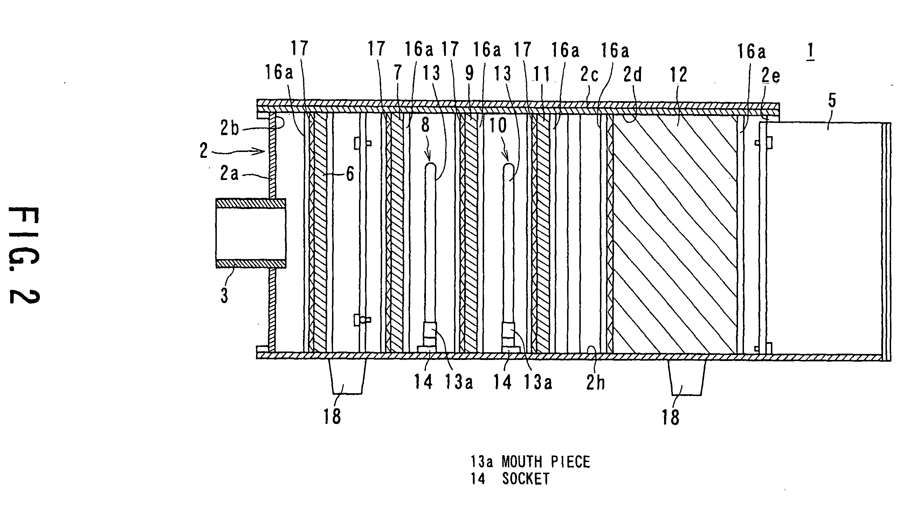

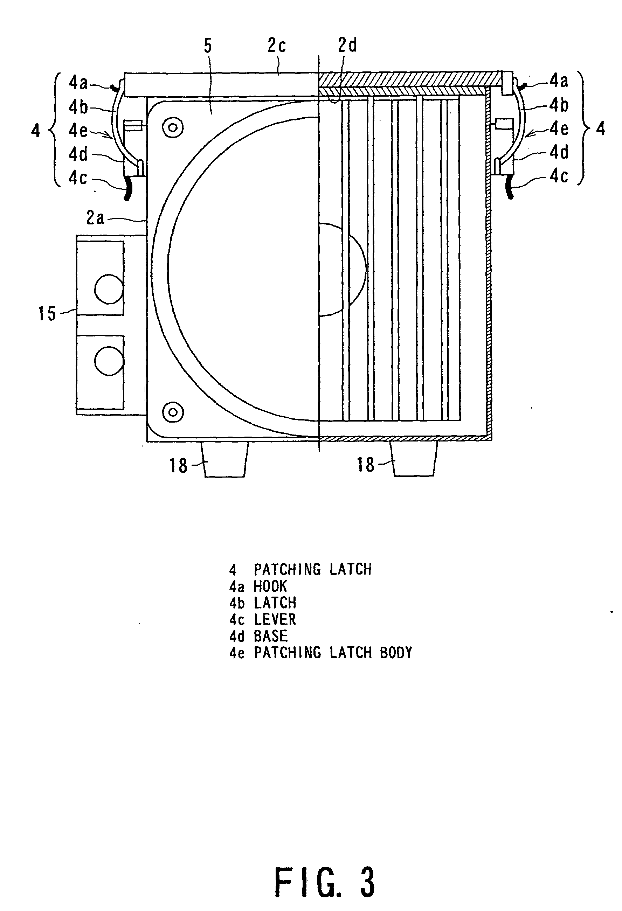

Fig. 1 is a plan view of a deodorization apparatus 1 according to the first embodiment of the present invention in which an upper lid, which is to be opened or closed, is removed from a case body, Fig. 2 is a sectional view taken along the line II-II in Fig. 1 in a state that the upper lid of the deodorization apparatus 1 is mounted to the case body, and Fig. 3 is a right side view, partially in section, of the deodorization apparatus shown in Fig. 2.

With reference to Figs. 1 to 3, the deodorization apparatus 1 has a bottomed cylindrical case body 2, rectangular in section, made of stainless steel and having one side surface (light side surface as viewed in Fig. 1) at the central portion of which an intake pipe 3, as short cylindrical pipe, for example, is secured to be substantially coaxial with the case body 2 in a manner so that inside and outside end portions of the intake pipe 3 slightly project inside and outside the case body 2.

To the outside end portion of the intake pipe 3, is connected one end of a suction hose, not shown, and the other one end of this suction hose is connected to a contaminated air source from which contaminated air containing odor gas is exhausted. As one example of such contaminated air source, there will be provided a grinding machine for grinding and working plastic lens for a spectacle disclosed, for example, in Japanese Patent Laid-open Publication No. HEI 6-226629. This machine generates much amount of hydrogen sulfide as sulfur containing odor component at the time of grinding and working the plastic lens, and the described embodiment thus aims to deodorize such hydrogen sulfide.

The case body 2 comprises a bottomed cylindrical body portion 2a rectangular in section and an upper lid 2c as an open/close lid in shape of rectangular plate for tightly closing an opened end 2b entirely opened at the upper end portion of the body portion 2a shown in Fig. 2. A packing 2d having a predetermined thickness is secured to an entire inner surface of the upper lid 2c.

The upper lid 2c is entirely pressed against the upper end portion 2b of the opening of the case body 2 by locking a plurality of patching latches 4, 4, ---, and the packing 2d of the upper lid 2c is pressed against the same so as to elastically deform the upper lid 2c to thereby air-tightly contact the opened upper end side.

That is, hooks 4a of the patching latches 4 are fixed to the respective corner portions of the outer side surfaces of the upper lid 2c, and patching latch bodies 4e, each composed of a ring-shaped latch 4b engageable with the hook 4a, a lever 4c to which the latch 4b is secured and a base 4d rotatably supporting one end of the lever 4c, are secured to the respective corner portions of the case body portion 2a of the opened upper end 2b side.

As shown in Figs. 1 and 2, the body portion 2a is provided with an exhaust port 2e formed to one axial end portion thereof opposite to the other axial end portion to which the intake pipe 3 is formed, i.e. right-hand end as viewed in Fig. 2, and an exhaust fan 5 is mounted to this exhaust port 2e. Inside the case body portion 2a, an air passage 2i for forcibly passing air in an arrow direction in the case body portion 2a so as to communicate with the intake pipe 3 and the exhaust fan 5.

Inside this air passage 2i, there are arranged, in the described order from the upstream side, intake pipe side, of the air flow towards the downstream side, exhaust fan side, thereof, with a predetermined distance, an air filter 6 for water removal, a first photo-catalyst filter 7, a first lamp unit 8, a second photo-catalyst filter 9, a second lamp unit 10, a third photo-catalyst filter 11 and an adsorbent unit 12.

The water removal air filter 6 is a filter having, for example, rectangular shape, which filtrates water content in the contaminated air and cut chips, dust or like which may be generated at a time of grinding and working plastic glass, for example. The air filter 6 has an outer edge trimmed by a rectangular outer frame member formed from metal or resin material so as to provide as a unit.

Further, each of the first, second and third photo-catalyst filters 7, 9 and 11 is provided with an air filter having a body formed from glass wool mesh. Both the outer surfaces of the air filter body in the air-flow direction are formed to show continuous waved shapes to thereby increase surface areas. Furthermore, a photocatalyst material and a fluorine resin material having good light resisting property is coated on the outer waved surfaces, and at least one of titanium oxide ($TiO_2$) having acidic surface and zinc oxide ($ZnO_2$) of amphoteric compound having high oxide gas adsorbing property, such as hydrogen sulfide. The outer peripheral edge portion of the filter having the structure mentioned above is trimmed and united by a rectangular outer frame formed from metal material or resin material.

The first and second lamp units 8 and 10 are examples of an ozone generator and include a plurality of ultraviolet lamps 13, each having a predetermined shape such as U-shape, these ultraviolet lamps 13 being arranged both sides in the axial central direction of the case body portion 2a as lateral pairs forming one set of lamp unit. Each of ultraviolet lamps 13 is one example of an ozone generator which irradiates ultraviolet rays mainly having wavelengths of about 185nm and 254nm and generates ozone around there and comprises a quartz glass bulb which has a U-shape through which the ultraviolet ray passes and in which a pair of electrodes are arranged and mercury and rare gases are sealed at a predetermined pressure.

As shown in Fig. 2, the ultraviolet (ray) lamp 13 is disposed in the case body portion 2a in a standing manner by plugging a single adopter 13a of the ultraviolet lamp 13 to a socket 14 disposed on an inner bottom surface 2h of the case body portion 2a. Each socket 14 is electrically connected to an inverter of a lighting apparatus, not shown, which is accommodated in an electrical part box 15 mounted on an outside surface of the case body portion 2a. Further, in this electrical part box 15, a power source for the exhaust fan 5 and the like are also accommodated.

The adsorbent unit 12 has a porous box shaped structure formed from a metal material or resin material, through which air flows in a direction of arrows, and a lot of impregnated carbon fills this porous box. The impregnated carbon is formed by applying an impregnating agent such as at least either one of iron oxide (FeO$_2$), potassium hydroxide, nickel nitrate or like to an outer surface of an activated carbon to thereby increase adsorbing power with respect to oxide gas or specific odor component. Further, in this embodiment, the impregnated carbon is formed by utilizing the potassium hydroxide.

As shown in Fig. 1, upper and lower pairs of guide rails 16a and 16b, each having a ⊐-shaped section, are secured to inner surfaces of a pair of upper and lower wall sections 2f and 2g of the case body portion 2a in states standing from the bottom surface 2h, and the water removal air filter 6, the first to third photo-catalyst filters 7, 9 and 11 and the adsorbent unit 12 accommodated in their outer frame structures are introduced into or taken out from the case body portion 2a through the opened upper end 2b of the case body portion 2a.

The water removal/air filter 6, the first to third photo-catalyst filters 7, 9 and 11 and the adsorbent unit 12 are elastically supported by interposing leaf plates as one example of a wave-shaped elastic member having protruded and recessed portions continuous in the height direction of the case body portion 2a between the respective guide rails 16a, 16b and the upstream side surfaces in the air flowing direction of the lateral pair of side end portions of the outer frame structures of the respective members of the water removal air filter 6, the first to third photo-catalyst filters 7, 9 and 11 and the adsorbent unit 12 to thereby prevent the respective units 6, 7, 9, 11 and 12 from rattling on the guide rails 16a, 16b by the contaminated air flow.

Furthermore, for example, four support rubbers 18, in forward tapered frustconical shape, as shown in Fig. 2, are secured to the respective four corner portions of the outer surface of the bottom portion 2h of the case body portion 2a, and the case body 2 can be settled by setting these support rubbers 18 on a desired floor or machine table. Further, specific ultraviolet lamps may be disposed near the first to third photo-catalyst filters 7, 9 and 11 for energizing photo-catalyst films of these photo-catalyst filters 7, 9 and 11.

Next, the function of the deodorization apparatus of the structure mentioned above will be descried.

At first, the upper lid 2c is mounted to the opened upper end 2b of the case body portion 2a and secured to be sealed by locking the respective patching latches 4 to make air tight the inside space of the case body portion 2a. The exhaust fan 5 is operated and the respective ultraviolet lamps 13 are lightened. Then, the air inside the case body 2 is exhausted outside by means of the exhaust fan 5 to thereby create a negative pressure state in the case body 2, so that the contaminated air containing odor component is sucked into the case body 2 from the intake pipe 3 through a suction hose, not shown.

The contaminated air introduced inside the case body 2 first passes the water removal air filter 6 to remove water component, cut chips, dust and the like, and thereafter, passes the first photo-catalyst filter 7 by which organic compound such as acetaldehyde (CH$_3$CHO) and nitride compound such as ammonium in the odor components in the contaminated air are adsorbed and oxidized and decomposed, as well as sulfide compound containing hydrogen sulfide (H$_2$S) is also oxidized and decomposed.

Thereafter, when the contaminated air flows through the first ultraviolet lamp unit 8 as the ozone generator, the contaminated air passes through an area in which ozone is generated by ultraviolet rays having wavelengths of 185nm and 254nm radiated from the two ultraviolet lamps 13. Accordingly, in this area, hydrogen sulfide (H$_2$S) in the contaminated air can be further oxidized and decomposed by the ozone to thereby be deodorized. On the further downstream side, organic compound such as acetaldehyde and nitride compound such as ammonium in the contaminated air are adsorbed and then oxidized and decomposed by the second photo-catalyst filter 9. Moreover, both the photo-catalyst films of the first and second photo-catalyst filters 7 and 9 disposed upstream side and downstream side of the downstream-side ultraviolet lamp 13 are energized and activated by this ultraviolet lamp 13 and the ozone generated, so that these first and second photo-catalyst filters 7 and 9 can preferably maintain the deodorization functions with respect to the organic compound and nitrogen compound in the contaminated air.

Thereafter, the contaminated air, which has been subjected to the deodorization treatment, is further oxidized and decomposed to thereby be deodorized by the ozone generated through the irradiation of the ultraviolet rays of the wavelength of about 185nm and about 254nm. Further, thereafter, organic compound such as acetaldehyde and nitrogen compound such as ammonium in the contaminated air are adsorbed by the third photo-catalyst filter 11 and then oxidized and decomposed to thereby be deodorized.

As mentioned above, the contaminated air is deodorized by the water removal air filter 6, the first to third photo-catalyst filters 7, 9, 11 and the first and second ultraviolet lamp units 8 and 10, in the described order, and the odor component is further adsorbed and deodorized by the activated carbon or impregnated carbon by passing through the adsorbent unit 12, and the air is thereafter discharged externally by means of exhaust fan 5.

Hereunder, the function and effect of the impregnated carbon will be described.

In the case where the impregnated carbon is formed by impregnating the activated carbon with potassium hydroxide (KOH), since the potassium hydroxide has an alkaline property, sulfur or like odor having acidic property will be easily captured as shown by the following equation (1).

$$H_2S + KOH \rightarrow K\text{-}HS + H_2 \qquad (1)$$

In the case of only the activated carbon, in an ozone atmosphere, this activated carbon (C) will be easily oxidized by the ozone ($O_3$) as shown by the following equation (2). This activated carbon is likely oxidized and deteriorated.

$$2O_3 + C \rightarrow 2O_2 + CO_2 \qquad (2)$$

However, in the present embodiment, potassium hydroxide exists on the activated carbon surface, so that the deterioration due to the oxidization of the activated carbon by the ozone can be substantially prevented or reduced by this potassium hydroxide, thus elongating a usable life time of the activated carbon.

Moreover, in the case where the sulfur odor component consists of hydrogen sulfide, the ozone and the hydrogen sulfide react, and the sulfur dioxide gas is generated as shown by the following equation (3) and sulfur is also generated as shown by the following equation (4).

$$H_2S + O_3 \rightarrow H_2O + SO_2 \qquad (3)$$

$$H_2S + O_3 \rightarrow S + H_2O + O_2 \qquad (4)$$

However, the sulfur dioxide gas and the sulfur are subjected, in the case body, to the deodorization treatment by the ozone, the photo-catalyst or the impregnated carbon, so that the external discharge of the sulfur odor component can be prevented or substantially reduced.

Furthermore, in a case where the impregnated carbon has a composition formed by impregnated activated carbon with iron oxide or nickel nitrate, sulfur odor component can be also effectively deodorized. That is, the iron oxide ($Fe_2O_3$) decomposes the hydrogen sulfide by desulfurizing function represented by the following equation (5).

$$Fe_2O_3 + 3H_2S \rightarrow Fe_2S_3 + 3H_2O \qquad (5)$$

Further, the nickel nitrate can promote the adsorption of the sulfur odor component to the activated carbon through its catalyst reaction and also can decompose the ozone into oxygen, so that the discharging of the odor component outside the case body can be prevented or substantially reduced.

Therefore, according to the deodorization apparatus of the characters mentioned above, since the contaminated air containing odor components introduced into the case body portion 2a is subjected to the deodorization treatment by the water removal air filter 6, the first to third photo-catalyst filters 7, 9, 11, the first and second lamp units 8, 10 and the adsorbent unit 12, that is, in seven stages, the odor components can be adequately deodorized through the one flow-pass in which the contaminated air taken from the intake pipe passes through the air-flow passage 2i and is discharged externally through the exhaust fan 5.

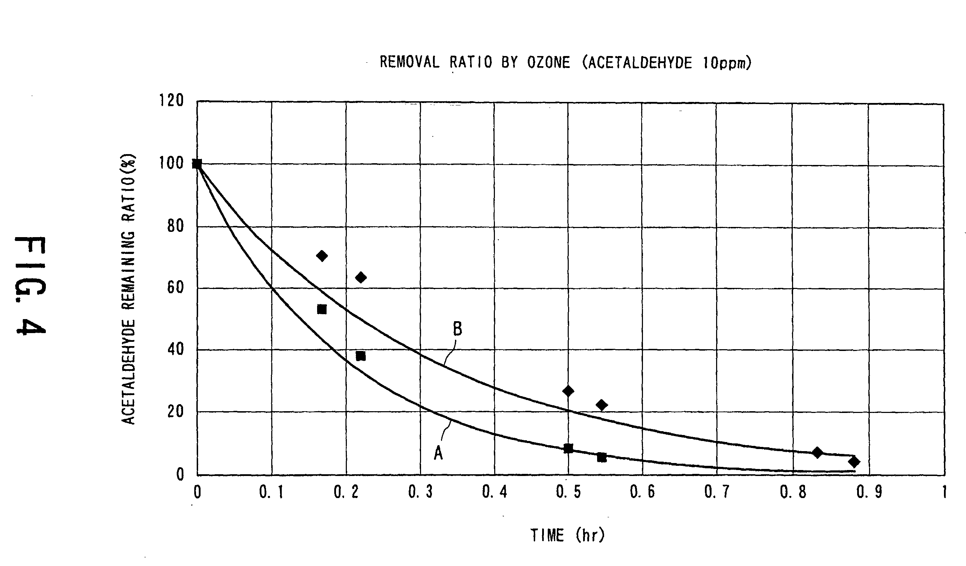

Fig. 4 is a graph representing a removal ratio of acetaldehyde by the ozone generated from the ultraviolet lamps 8 and 10 as one example of an ozone generator. That is, in the graph, the curves A and B represent remaining ratio of acetaldehyde remaining in a sealed container having an inner volume of 1 mm$^3$ in which a photo-catalyst film and three ultraviolet lamps 8, 10 and three bactericidal lamps are disposed, at a time when acetaldehyde having odor component is injected with a predetermined density (for example, of 10 ppm). Actually, in the graph, the curve A represents the acetaldehyde remaining ratio (%) in the case of three bactericidal lamps which generate the ozone and three photo-catalyst excitation lamps which excite the photo-catalyst films, and on the other hand, the curve B represents the acetaldehyde remaining ratio (%) in the case of three bactericidal lamps which do not generate ozone and three photo-catalyst excitation lamps which excite the photo-catalyst films. With reference to Fig. 4, it will be found that the acetaldehyde of odor component can be removed with high removal ratio in the case of the curve A more than that in the case of the curve B.

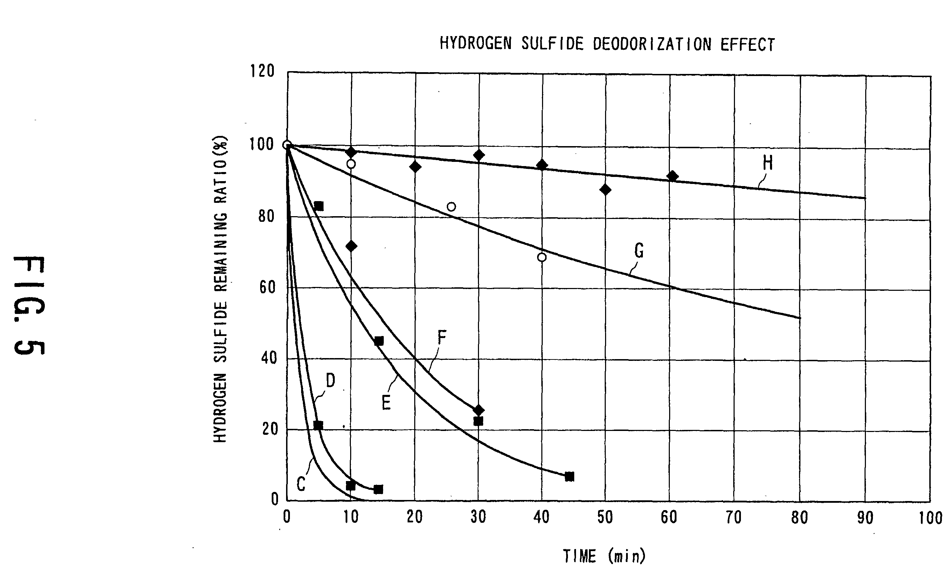

Fig. 5 is a graph in which curves C and D represent deodorization effect in the case of using the deodorization apparatus 1 of this invention which is disposed in a sealed container having an inner volume of 1 mm$^3$ in which the hydrogen sulfide ($H_2S$), which may be exhausted at a grinding working time of plastic lenses by using a grinding machine, is injected at a predetermined density, and the graph also includes curves E, F, G and H showing deodorization effect by using other deodorization means.

That is, the curves C and D represent deodorization effects of the odor component containing the hydrogen sulfide ($H_2S$) achieved in functional combination of ozone generated by the first and second lamp units 8, 10, the photo-catalysts of the first to third photo-catalyst filters 7, 9, 11 and the activated carbon (or impregnated carbon) in the adsorbent unit 12 as in the deodorization apparatus 1 of the present invention, and air flow velocities in the cases C and D are 4.5 m/s and 3.5m/s, respectively.

On the other hand, the curve E represents deodorization effect achieved in functional combination of the first and second lamp units 8, 10 for the ozone generation and the photo-catalysts of the first to third photo-catalyst filters 7, 9, 11, the curve F represents deodorization effect achieved only by the first and second lamp units 8, 10 for the ozone generation, the curve G represents deodorization effect achieved in functional combination of the photo-catalysts of the first to third photo-catalyst filters 7, 9, 11 and an ultraviolet lamp which irradiates ultraviolet rays for exciting the photo-catalyst films, and the curve H represents reduction of the hydrogen sulfide through natural damping without providing any deodorization means.

As shown in Fig. 5, the most effective deodorization effect (curve C, D) can be achieved by using the deodorization apparatus of the present invention and the density of the hydrogen sulfide can be remarkably reduced for the shortest time in comparison with the other deodorization means.

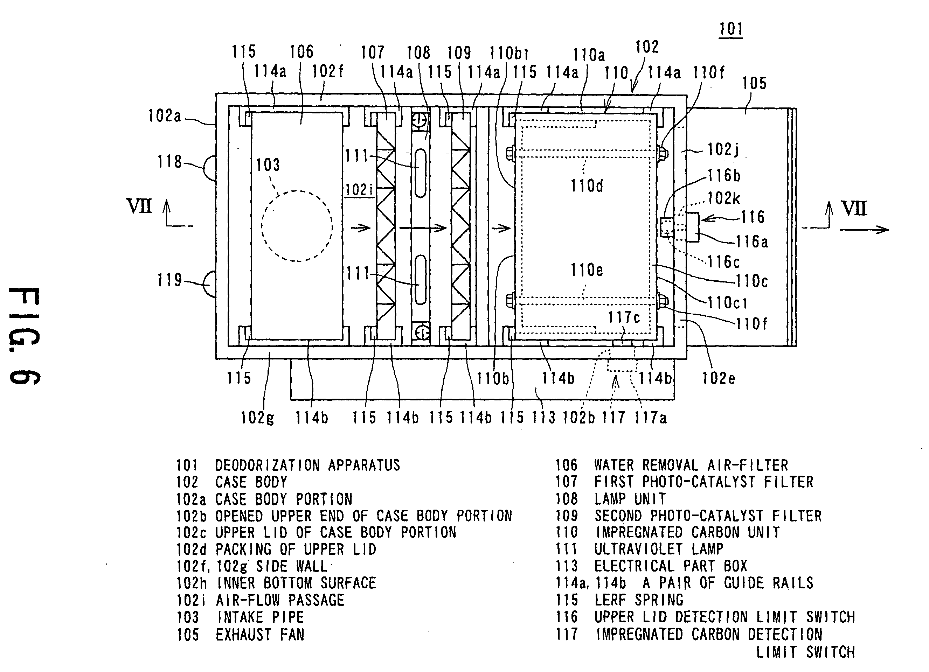

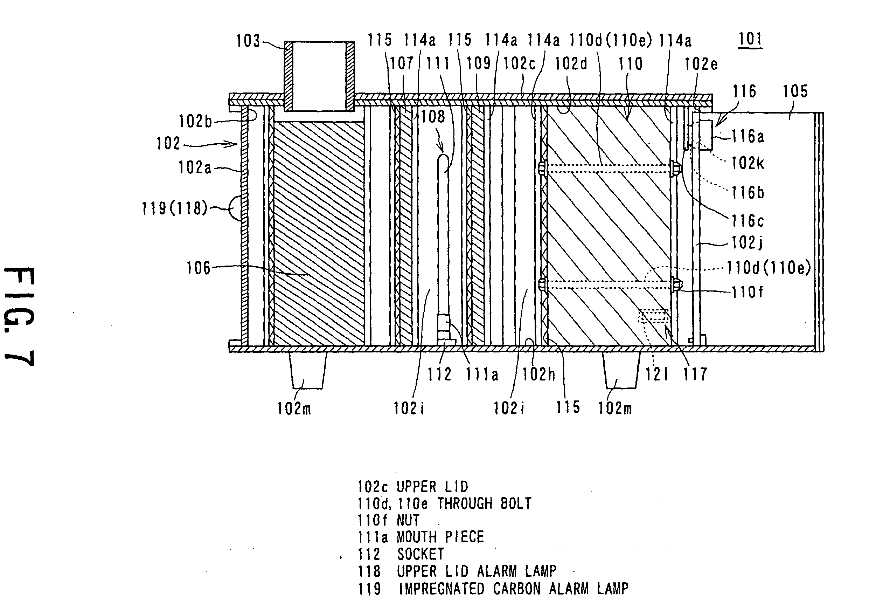

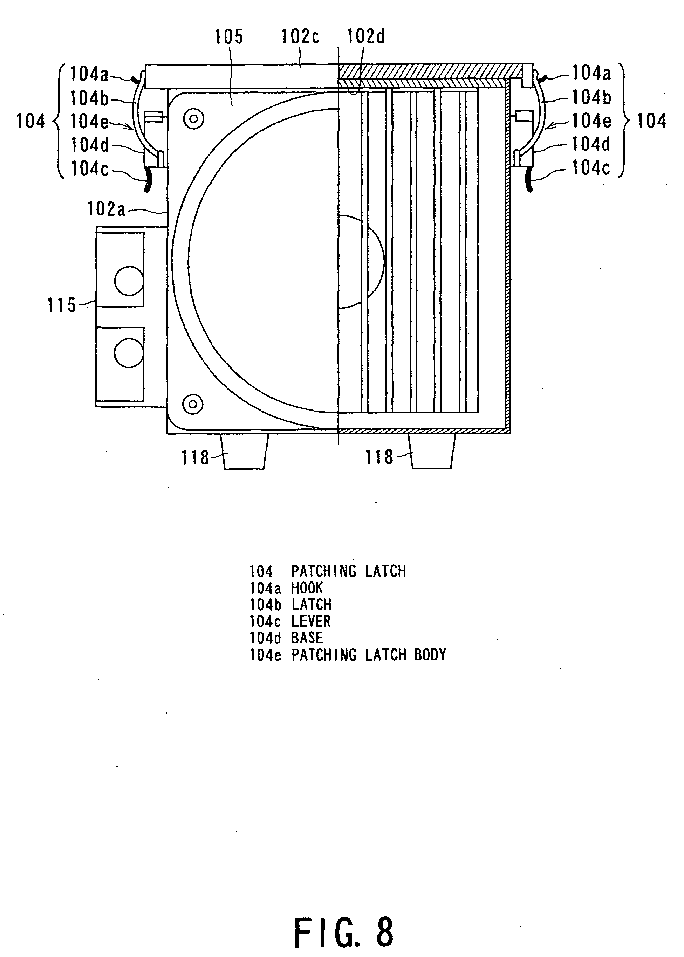

Fig. 6 is a plan view of a deodorization apparatus 101 according to the second embodiment of the present invention in a state that an upper lid 102c, which is opened or closed, is removed from a case body portion 102a, Fig. 7 is a sectional view taken along the line VII-VII in Fig. 6 in a state that the upper lid 102c is mounted to the case body portion 102a, and Fig. 8 is a right-side view of Fig. 7 partially in section.

As shown in Figs. 6 and 7, the deodorization apparatus 101 is provided with a case body 102 made from a stainless steel and having a bottomed cylindrical shape rectangular in section. The case body 102 has a case body portion 102a having a bottomed cylindrical shape rectangular in section and having an opened upper end 102b to which the upper lid 102c in form of rectangular plate is mounted to be opened or closed, and a packing 102d having a predetermined thickness is fixedly applied to an entire inner surface of this upper lid 102c.

The upper lid 102c is provided with an intake pipe 103 having, for example, a short cylindrical pipe structure at a left end portion, as viewed, so as to penetrate in the direction of the plate thickness, and inner and outer end portions of the intake pipe 103 project slightly inward and outward, respectively, from the case body 102.

To the outer end portion of the intake pipe 103, is connected one end of an intake hose, not shown, which has another end to which a contaminated air source for exhausting contaminated air containing odor gas is connected. Such contaminated air source will include, as one example, a grinding machine for grinding plastic lens for spectacles or like as disclosed in Japanese Patent Laid-open Publication No. 226629/1994. This grinding machine generates much hydrogen sulfide as sulfur odor component contained in the plastic lens at the time of grinding it. This embodiment, therefore, also aims to deodorize the hydrogen sulfide.

As shown in Fig. 8, the upper lid 102c is entirely pressed against the opened upper end portion 102b of the case body 102 by locking a plurality of patching latches 104, 104, ---, and the packing 102d of the upper lid 102c is pressed against the same so as to elastically deform the upper lid 102c to thereby air-tightly contact the opened upper end side 102b.

That is, hooks 104a of the patching latches 104 are fixed to the respective corner portions of the outer side surfaces of the upper lid 102c, and patching latch bodies 104e, each composed of a ring-shaped latch 104b engageable with the hook 104a, a lever 104c to which the latch 104b is secured and a base 104d rotatably supporting one end of the lever 104c, are secured to the respective corner portions of the case body portion 102a on the opened upper end 102b.

As shown in Figs. 6 and 7, the case body portion 102a is provided with an exhaust port 102e formed to one end portion thereof on the opposite end portion of the intake pipe 103, i.e. right-hand end as viewed in Fig. 7, and an exhaust fan 105 as one example of air-flowing apparatus is mounted to this exhaust port 102e from the outside of the case body portion 102a. The exhaust fan 105 has an inner diameter of, for example, 145 mm. Inside the case body portion 102a, an air passage 102i for forcibly passing air in an arrow direction in the case body portion 102a so as to communicate with exhaust fan 105 and the intake pipe 103.

Inside this air passage 102i, there are arranged a water removal air filter 106, a first photo-catalyst filter 107, a first lamp unit 108, a second photo-catalyst filter 109, and an impregnated carbon unit 110 in the described order with predetermined distance each other from the upstream side, on an intake pipe side, of the air flow towards the downstream side, on an exhaust fan side, thereof.

As shown in Fig. 7, the water removal air filter 106 is a filter having, for example, rectangular shape, which filtrates water content in the contaminated air, which is sucked from the intake pipe 103 towards the case body 102, and cut chips, dust or like which may be generated at a time of grinding plastic lens, for example. The air filter 106 has outer edges which are trimmed by a rectangular outer frame member formed from metal or resin material so as to be formed as a unit.

Further, each of the first and second photo-catalyst filters 107 and 109 is provided with an air filter having a body formed from glass wool mesh. Both the outer surfaces of the air filter body in the air-flow direction are formed to show continuous wave-shaped configuration to thereby increase surface areas. Furthermore, a photo-catalyst material and a fluorine resin material having good light-proof property is coated on the outer waved surfaces, and at least one of titanium oxide ($TiO_2$) having acidic surface and zinc oxide ($ZnO_2$) of amphoteric compound having high oxide gas adsorbing property, such as hydrogen sulfide. The outer peripheral edge portion of the filter having the structure mentioned above is trimmed and united by a rectangular outer frame formed from metal material or resin material.

The lamp unit 108 is an example of an ozone generator and includes a plurality of ultraviolet lamps 111, each having a predetermined shape such as U-shape, these ultraviolet lamps 111 being arranged both sides in the axial central direction of the case body portion 102a as lateral pairs forming one set of lamp unit. The number of the lamps to be arranged is not limited. Each of ultraviolet lamps 111 is one example of an ozone generator which irradiates ultraviolet rays mainly having wavelengths of about 185nm and 254nm and generates ozone around there and comprises a quartz glass bulb which has a U-shape through which the ultraviolet ray passes and in which a pair of electrodes are arranged and mercury and rare gases are sealed at a predetermined pressure.

As shown in Fig. 7, the ultraviolet ray lamp 111 is disposed in the case body portion 102a in a standing manner by plugging an electricity receiving pin, to be detachably, to each socket 112 disposed on an inner bottom surface 102h of the case body portion 102a. Each socket 112 is electrically connected to an inverter of a lighting apparatus, not shown, which is accommodated in a rectangular cylindrical electrical part box 113 mounted on an outside surface of the case body portion 102a. Further, in this electrical part box 113, electrical parts including a power source for the exhaust fan 105 and the like are also accommodated.

The adsorbent unit 110 has a porous box-shaped structure formed from a metal material or resin material, through which air flows in a direction of arrows, and a lot of impregnated carbon, not shown, fills this porous box. The impregnated carbon is formed by applying an impregnating agent such as at least either one of iron oxide ($FeO_2$), potassium hydroxide, nickel nitrate or like to an outer surface of a pellet type impregnated carbon in form of small column having a grain diameter of 4 mm to 6 mm and a length of 6 mm to thereby increase adsorbing power with respect to oxide gas or specific odor component. Further, in this embodiment, the impregnated carbon impregnated with potassium hydroxide fills the rectangular cylindrical unit case 110a.

The rectangular cylindrical unit case 110a of the impregnated carbon unit 110 has a structure capable of adjusting its thickness in the air-flow direction. That is, the porous rectangular cylindrical unit case 110a is composed of porous rectangular cylindrical inner box 110b having one end opening and a porous rectangular cylindrical outer box 110c having a slightly larger size than that of the inner box and having an end opening to which the opened end portion of the inner box 110b is tightly fitted in a telescopical manner, the inner and outer boxes 110b and 110c being opposed and fitted in the air-flow direction shown in Fig. 6 with arrows.

These inner and outer boxes 110b and 110c have opposed plates 110b1 and 110c1, between which a pair of lateral through bolts for adjustment 110d and 110e are inserted at upper and lower two stages as shown in Fig. 7, and nuts 110f, 110f are fastened to the inserted end portions.

Accordingly, by moving the inner box 110b in the air-flow direction in the outer box 110c, the thickness of the unit case 110a in the air-flow direction can be easily and smoothly adjusted, and the thickness of the unit case 110a can be secured by a plurality of through bolts 110d, 110e and nuts 110f, 110f.

Furthermore, according to the unit case 110a, it is not necessary to preliminarily prepare a plurality of unit cases having different sizes, so that the cost reduction can be achieved.

Then, in the present impregnated carbon unit 110, the thickness in the air-flow direction is set to be 70 mm to 130 mm at air-flow rate of 10 $m^3$/hr to 20 $m^3$/hr.

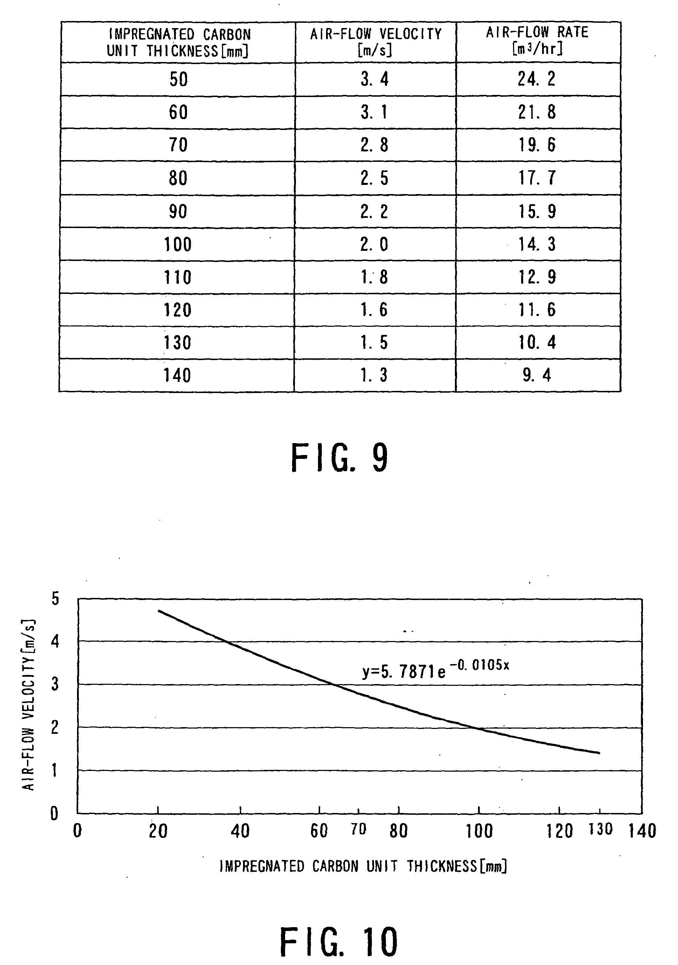

Fig. 9 is a table of data based on results of experiment showing changes or variations of the air-flow rate inside the case body 2 at a time of variously changing the thickness of the impregnated carbon unit 10 to obtain optimum value of the thickness in the air-flow direction. Fig. 10 is a graph showing the results of the experiment mentioned above.

In this experiment, it was found that high deodorization effect could be obtained at a time of using the impregnated carbon unit 110 having its thickness of 70 mm to 130 mm in the air-flow direction with high efficiency at the air flow rate of 10$m^3$/hr to 20$m^3$/hr.

In the case of the thickness of the impregnated carbon unit 110 of less than 70 mm, the air-flow resistance is reduced and the air-flow rate is increased to 21.8$m^3$/hr, but the impregnated amount of the impregnated carbon is reduced, so that the odor component in the exhaust gas from the exhaust fan 5 is increased, and hence, sufficient deodorization effect could not be achieved.

On the other hand, in the case of the thickness of the impregnated carbon unit 110 of more than 130 mm, there caused an inconvenience such that odor component in an exhaust gas leaking from, for example, a grinding machine, as a contaminated air generation source, connected to the intake pipe 103 of the deodorization apparatus 101. This is because pressure loss is increased by the increasing of the thickness of the impregnated carbon unit 110 in the air-flow direction and suction force by means of the exhaust fan 105 is lowered, thus reducing the deodorizing amount per predetermined time.

As shown in Fig. 6, a plurality of upper and lower pared guide rails 114a and 114b, each having a ⊐-shaped section, are secured to inner surfaces of a pair of upper and lower wall sections 102f and 102g of the case body portion 102a. These guide rails 114a and 114b are guide rails for guiding, to be detachably, the insertion of left and right side end portions in Fig. 6 (up- and down-stream sides in the air-flow direction) of the outer frames of the water removal air filter 106, the first and second photo-catalyst filters 107, 109 and the impregnated carbon unit 110 from the opened upper end portion 102b of the case body 102a onto the inner bottom surface 102h thereof.

The water removal air filter 106, the first and second photo-catalyst filters 107, 109 and the impregnated carbon unit 110 are elastically supported by interposing leaf plates 117 as one example of a wave-shaped elastic member having protruded and recessed portions continuous in the height direction of the case body portion 102a between the respective guide rails 114a, 114b and the upstream side surfaces in the air flowing direction of the lateral pair of side end portions of the outer frame structures of the respective members of the water removal air filter 106, the first and second photo-catalyst filters 107, 109 and the impregnated carbon unit 110 to thereby prevent the respective members and units 106, 107, 109 and 110 from rattling on the guide rails 114a, 114b by the contaminated air flow.

Furthermore, for example, four support rubbers 116, in forward tapered frustconical shape, as shown in Fig. 6, are secured to the respective four corner portions of the outer surface of the bottom portion 102h of the case body portion 102a, and the case body can be settled by setting these support rubbers 116 on a desired floor or machine table. Further, specific ultraviolet lamps may be disposed near the first and second photo-catalyst filters 107 and 109 for energizing photo-catalyst films of these photo-catalyst filters independent from the ultraviolet lamp 111 commonly utilized as ozone generator.

As shown in Figs. 6 and 7, a limit switch 116 for detection of the upper lid as one example of first detection means and a limit switch 117 for detection of the impregnated carbon unit as one example of second detection means are disposed on the downstream side in the air-flow direction of the impregnated carbon unit 110. Further, upper lid alarm lamp 118 and an impregnated carbon alarm lamp 119 are also disposed, for example, in front of the case body portion 102a.

The upper lid alarm lamp 118 is an alarm lamp for alarming, through lighting or flashing of the alarm lamp, a fact of erroneous mounting of the upper lid 102c, for example of shifting of the upper lid 102c from the opened upper end 102b of the case body portion 102a or non-mounting of the upper lid 102c to the opened upper end portion 102b. The impregnated carbon alarm lamp 119 is an alarm lamp for alarming a fact of no-insertion of the impregnated carbon unit 110 into a predetermined portion in the case body portion 102a upon the detection of an occurrence that the leak amount of the ozone leaking to the external portion from the opened upper end portion of the case body portion 102a exceeds an allowable limit.

The limit switch 116 for the detection of the upper lid is a kind of safety device for driving a necessary alarming operation such as lightening the alarm lump at the time of detecting the opened state of the opened upper end 102b for the reason of the shifting of the upper lid 102c from the opened upper end 102b of the case body portion 102a or of the non-mounting of the upper lid 102c to the opened upper end portion 102b, or otherwise, for extinguishing the lighting (stopping the operation) of the ultraviolet lamp 111, which generates the ozone, to thereby stop or prevent the generation of the ozone. The limit switch 117 is provided with a first micro-switch 116a and an operation pin 116b for switching and controlling the operation of the micro-switch 116a.

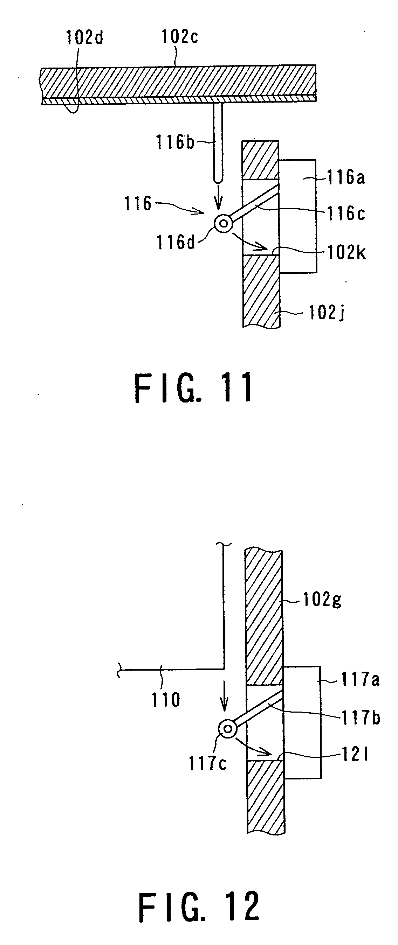

As shown in Fig. 11, the first micro-switch 116a includes a switching point, not shown, and a swingable lever 116c for switching and controlling this switching point and a rotatable roller 116d mounted to the free front end portion of this lever 116c.

The lever 116c swings between an operation block/stop position at which the roller 116d projects inward the case body portion 102a and an operation position at which the lever is swung downward in Fig. 9 to thereby switch the switching point, and the lever 116c is urged by a return spring or like to the operation block/stop position.

As shown in Figs. 6, 7 and 11, the first micro-switch 116a is attached to the outer surface of the upper end, in the drawing, of the back plate 102j at the right end, in Fig. 6 or 7, of the case body 102 downstream side of the impregnated carbon unit 110 in the air-flow direction, and at the mounting surface side of this first micro-switch 116a, a first small hole 102k penetrating in the plate thickness direction is formed to the back plate 102j. The lever 116c of the first micro-switch 116a is inserted into this first small hole 112k so that the roller 116d slightly projects into the case body portion 102a.

The operation pin 116b positioned above the projection of the roller 116d is disposed so as to project downward in Fig. 9 at the inner surface of the back surface side end portion of the upper lid 102c so as to strongly press the upper lid 102c against the opened upper end portion 102b of the case body portion 102a, and when the upper lid 102c is locked by the patching latch 104, the operation pin 116b abuts against the roller 116d to swing the lever 116d and push it into the small hole 102k and then to switch the switching point containing the micro-switch 116a from the operation block/stop side contact to the first operation side contact.

That is, this switching contact operates to drive the alarm means such as upper lid alarm lamp 118 of an electricity supply side circuit connected to a power source and to switch and control two contacts between the operation block/stop contact for blocking or stopping the lightening of the ultraviolet lamp 111 and the first operation side contact for lightening the ultraviolet lamp 111, 111 for generating the ozone, and this switching contact is always urged by the return spring or like so as to be switched to the operation block/stop side contact.

On the other hand, the limit switch 117 for the impregnated carbon detection is a safety device, which drives the required alarm means for lighting or flashing the impregnated carbon alarm lamp 119 at the time when the impregnated carbon unit 110 is not inserted into the predetermined portion of the case body portion 102a to thereby detect the fact that there causes a state that the leak amount of the ozone leaking externally from the case body portion 102a exceeds the allowable limit, and at this time, an alarm is generated for alarming the no-insertion of the impregnated carbon unit 110 and, at the same time, the lightening (operation) of the ultraviolet lamps 111, 111, which generate the ozone, are extinguished (stopped), or this limit switch 117 for the impregnated carbon unit detection acts to preliminarily block the lightening thereof. Such safety device includes a second micro-switch.

As shown in Fig. 12, the second micro-switch 117a includes a switching contact, now shown, and is provided with a swingable lever 117b adapted to switch and control the switching contact and a rotatable roller 117c mounted to the free front end of the lever 117b.

The lever 117b swings between an operation block/stop position at which the roller 117c projects inward the case body portion 102a and a second operation position at which the lever is swung rightward in Fig. 12 to thereby switch the switching point, and the lever 116c is urged always by a return spring or like to the operation block/stop position.

As shown in Figs. 6, 7 and 12, the second micro-switch 117a is accommodated in an electrical part box 113 and attached to the lower portion of an outer surface of one side wall, for example, 102g, of the case body portion 102a opposing, with space, to the outer surface of the downstream side end of the impregnated carbon unit 110 in the air-flow direction.

That is, as shown in Fig. 12, the case body portion 102a is formed, at the mounting surface of the one side wall 102g to which the second micro-switch 117a is attached, with a second small hole 121 penetrating in a plate thickness direction. The lever 117b of the second micro-switch 117a is inserted into this second small hole 121 so that the roller 117c projects slightly into the case body portion 102a. That is, the roller 117c projects into the insertion passage at the time of inserting the impregnated carbon unit 110 into the case body portion 102a under the guidance of the guide rails 114a, 114b.

Accordingly, when the impregnated carbon unit 110 is inserted into the case body portion 102a, the outer bottom surface of the one side end portion of the impregnated carbon unit 110 abuts against the roller 117c and pushes it outward and the lever 117b is swung sideways so as to push it into the second small hole 121 to thereby switch the switching contact of the second micro-switch 117a to the second operation side contact from the operation block/stop side contact.

That is, this switching contact operates to drive (lighten) the alarm means such as impregnated carbon alarm lamp 119 of an electricity supply side circuit connected to a power source and to switch and control two contacts between the operation block/stop contact for blocking or stopping the lightening of the ultraviolet lamp 111 and the second operation side contact for lightening the ultraviolet lamps 111, 111 for generating the ozone, and this switching contact is always urged by the return spring or like so as to be switched to the operation block/stop side contact.

Furthermore, the second operation side contact of this second micro-switch 117a is connected electrically in series to the first operation side contact of the first micro-switch 116a in a manner such that both the first and second switching contacts of the both the first and second micro-switches 116a and 117a are respectively switched to the first and second operation side contacts, the power from the power supply side circuit is supplied to the ultraviolet lamps 111, 111 to thereby enable to be lightened (operated), and in the case other than the above case, the lightening of the ultraviolet lamps 111, 111 is blocked or stopped. Further, the upper lid alarm lamp 118 and the impregnated carbon alarm lamp 119 may be mounted to portions other than the front surface of the case body portion 102a.

Next, the use and function of the deodorization apparatus 101 of the structure mentioned above will be described.

A power switch, not shown, is first switched on. Then, it is confirmed whether the upper lid alarm lamp 118 and the impregnated carbon alarm lamp 119 disposed at the front surface of the case body portion 102 are lightened or flashed to thereby generate an alarm.

For example, supposing the case that the impregnated carbon alarm 119 is lightened or flashed, it is found that the impregnated carbon unit 110 is not inserted into the predetermined position in the case body 102, and at this time, the impregnated carbon unit 110 is inserted into the predetermined position.

Then, as shown in Fig. 12, the outer bottom surface of the impregnated carbon unit 110 abuts against the roller 117c of the second micro-switch 117a, depresses it downward as viewed in Fig. 12 and pushes it outward, and the switching contact of the micro-switch 117a is switched from the operation block/stop side contact to the second operation side contact. According to this switching operation, the lightening or flashing of the impregnated carbon alarm lamp 118 is extinguished, and the ultraviolet lamps 111, 111 become operative (capable of lightening).

Furthermore, at this time, in addition to the above state, in case an alarm is generated by lightening or flashing the upper lid alarm lump 118, the upper lid 102c is covered over the opened upper end 102b of the case body portion 102a and then patching latches 4 are locked.

Then, as shown in Fig. 11, when the upper lid 102c is placed on the opened upper end 102b of the case body portion 102a, the operation pin 116b of the upper lid 102c abuts against the roller 116d of the limit switch 116 and pushes it on the side of the first small hole 102k and then swing the lever 116d to thereby switch the switching contact housed in the micro-switch from the operation block/stop contact side to the first operation side contact.

For this reason, the lightening or flashing of the upper lid alarm lamp 118 is extinguished, and the first operation side contact is connected in series to the second operation side contact and also connected to the power supply side circuit, so that the power is supplied to the ultraviolet lamps 111, 111, which are then lightened (driven).

Accordingly, the exhaust fan 105 is driven in accordance with the lightening of the ultraviolet lamps 111, 111, and the air inside the case body 102 is exhausted outward by the operation of the exhaust fan 105 to thereby create a negative pressure state in the case body 102. Thus, the contaminated air containing odor component is sucked from the intake pipe 103 through an intake hose, not shown, into the case body 102.

The contaminated air introduced into the case body 102 first passes the water removal air filter 106 to thereby remove water content, chip, dust or like and then passes the first photo-catalyst filter 107 in which an organic component such as acetaldehyde $(CH_3CHO)$ in the odor component and nitride compound such as ammonium are adsorbed and oxidized and decomposed. That is, a sulfur odor component including sulfur hydride $(H_2S)$ is also oxidized and decomposed.

Thereafter, at the time when the contaminated air passes the ultraviolet lamp unit 108 as ozone generator, the contaminated air passes through the area in which the ozone is generated by ultraviolet rays having wavelengths of 185nm and 254nm irradiated from the ultraviolet lamps 111, 111, and accordingly, the hydrogen sulfide in the contaminated air can be further oxidized and decomposed, thus being deodorized. Furthermore, on both the upstream and downstream sides thereof, the photo-catalyst films of the first and second photo-catalyst filters 107, 109 are exited by the ultraviolet rays irradiated from the ultraviolet lamps 111, 111, so that the organic compound such as acetaldehyde and the nitride compound such as ammonium in the contaminated air are adsorbed and then oxidized and decomposed. Moreover, since the photo-catalyst films of both the first and second photo-catalyst filters 107, 109 are excited and activated by the ultraviolet rays and ozone from the ultraviolet lamps 111, 111, the deodorizing function of the first and second photo-catalyst filters 107, 109 with respect to the organic compound and the nitride compound can be effectively maintained.

As mentioned above, the contaminated air is deodorized in order by the water removal air filter 106, the first and second photo-catalyst filters 107, 109 and the ultraviolet lamp unit 108, and then, passes through the impregnated carbon unit 110 in which the odor component in the contaminated air is adsorbed by the impregnated carbon, which is thereafter deodorized and exhausted externally through the exhaust fan 105.

On the other hand, in the case where the upper lid 102c is not mounted on the opened upper end 102b of the case body portion 102a, the upper lid alarm lamp 118 is lightened or flashed to alarm the fact that the upper lid 102c is not mounted in position and to prevent the lightening (operation) of the ultraviolet lamps 111, 111, so that the ozone is generated through the lightening of each ultraviolet lamp 111 and the external leak through the opened upper end 102b of the case body portion 102a can be prevented.

Furthermore, during the operation of the deodorization apparatus 101, i.e., during the lightening of the ultraviolet lamps 111, 111, when the upper lid 102c is removed by some reason from the case body portion 102a, such removal of the upper lid 102c is detected by the upper lid detection limit switch 116 and the power supply to the ultraviolet lamps 111, 111 now lightening is stopped and forcibly extinguished, so that the ozone can be prevented or reduced from leaking outward from the body case 102, thus improving safeness function to a human body.

Furthermore, in the case of no insertion of the impregnated carbon unit 110 to the predetermined position in the case body portion 102a because of, for example, exchanging of the impregnated carbon unit 110, the impregnated carbon alarm lamp 119 is lightened or flashed to alarm the no-insertion of the impregnated carbon unit 110 and the lightening (operation) of the respective ultraviolet lamps 111 is prevented, thereby preventing the ozone from being generated from the ultraviolet lamps 111. Thus, the adsorption of the ozone by the by the impregnated carbon can be prevented, and the leaking of the ozone outward the case body 102 can be also prevented or effectively restricted.

That is, the ultraviolet lamps for generating the ozone can be lightened (operated) only upon the confirmation of the fact that the impregnated carbon unit 110 is inserted and placed to the predetermined position in the case body portion 102a and the upper lid 102c is mounted to the opened upper end 102b of the case body portion 102a. Therefore, the fear of the ozone leaking to the external portion of the body case 102 can be extremely reduced and the safeness to the human body can be improved.

Further, in the embodiments described above, there are described examples of the ultraviolet lamps 8, 10 and 111 as one example of the ozone generator, the present invention is not limited to this example and, as an ozone generator, there may be adopted a device of, for example, metal fine wire seal type, metal oxide powder seal type, diffusion-drift type, nitrogen discharge light silent discharge superimposing type, rotating electrode type, cryogenic operative glow discharge type, double-discharge type, corona discharge type, electrolytic method type or like.

The metal fine wire seal type ozone generator has a double-tube structure comprising an outer tubular glass container for passing material gas and an inner stainless tube disposed inside the outer glass container for passing cooling air so that one end of an inner electrode is air-tightly accommodated therein, and an outer electrode opposing, in a diametrical direction, to the inner electrode is disposed to the outer surface of the container in the manner such that a metal fine wire is disposed inside the glass container between these outer electrode and inner electrode.

The metal oxide powder seal type ozone generator has a structure in which various type oxides fill a silent discharge generation space.

The dispersion drift type ozone generator is a generator having a structure for increasing quantity of generated ozone by possibly avoiding destroy of ozone generated by successive discharge considered to be one factor of reduction of ozone generation efficiency and quickly drifting the generated ozone from discharge field to non-discharge field.

The nitrogen discharge light silent discharge superimpose type ozone generator is an ozone generator for generating the ozone through the nitrogen discharge light to oxygen silence discharge.

The rotating electrode type ozone generator has a structure in which ozone is generated by ionization through the discharge between a plurality of earth side line electrodes embedded in a surface of a columnar rotary body made of, for example, vinyl chloride, and a high pressure side electrode formed from, for example, a copper rod, through the rotation of a disc-shaped electrode.

The cryogenic operative glow discharge type (or creeping discharge type) ozone generator, which is dipped in liquid nitrogen and the generates the ozone by the glow discharge of low gas pressure of 0.5 to 2 Torr or high frequency creeping discharge. Further, the creeping discharge type ozone generator is of a type which performs no forcible cooling and in which an alternative current (A.C) (5.5 kV) is applied between a $CuSO_4$ solution in a glass tube and a stainless wire wound up around an outer periphery of the glass tube to thereby generates creeping discharge on the outside of the glass tube and then to generate the ozone in the air or oxygen.

The double-discharge type ozone generator is a in which a preliminary discharge is first effected to supply a lot of initial electrons near a cathode and, according thereto, a main discharge is then performed.

The corona discharge type ozone generator is one for generating the ozone by positive streamer pulse corona in the air. This type generator specifically includes a cathode of a shape of approximate Rogowskii (plate electrode) or ball electrode, in which four to six anodes, each in blade shape, are disposed and a gap length is 10 to 25 mm.

The electrolytic method type ozone generator includes one practical example of water electrolytic type ozone generator utilizing an ion exchanging resin film functioning as a solid electrolyte in place of an electrolyte in water. In such ozone generator, the quantity of ozone of 0.2 to 120 g/hr will be generated.

Furthermore, in the respective embodiments mentioned above, both the side surfaces of the filter body of each of the first to third photo-catalyst filters 7, 9, 11, 107, 109 have the vertically continuous waved-shape in the air-flow direction. The present invention is, however, not limited to such embodiments and the filter bodies of the first to third photo-catalyst filters 7, 9, 11, 107, 109 may have waved bent surfaces formed along the air-flow direction. According to such modification, the contacting area between the contaminated air and the first to third photo-catalyst filters 7, 9, 11, 107, 109 is increased to thereby increase the deodorization effect, as well as the reduction of the air-flow resistance in the case body portions 2a, 102a, thus reducing a pressure loss.

Furthermore, in the above embodiments, the U-shaped first and second lamp units 8, 10, 108 are disposed in a standing manner such that the axis of the straight tube portion thereof directs to a perpendicular direction with respect to the air-flow direction, but these first and second lamp units 8, 10, 108 may be horizontally disposed in a manner such that the axis of the straight tube portion thereof directs substantially to a parallel direction with respect to the air-flow direction. According to such arrangement, the air-flow resistance in the case body 2a, 102a can be reduced and, therefore, the pressure loss can be also reduced. Accordingly, in the arrangement in which the first and second lamp unit 8, 10, 108 are horizontally disposed, and the first to third photo-catalyst filters are formed so that the bent direction of the bent surfaces of the filter bodies thereof are substantially parallel to the air-flow direction, the more improved reduction of the pressure loss can be achieved.

Industrial Usage

As mentioned above, according to the present invention, when the contaminated air containing odor gas passes through an air passage formed in the body case, the odor component is oxidized and decomposed by ozone generated by the ozone generator to be thereby deodorized, then further oxidized and decomposed by the photo-catalyst filters and further deodorized through the adsorption of the impregnated carbon. Accordingly, through one-pass flowing of the contaminated air in the air-flow passage in the case body, the odor component in the contaminated air is deodorized three times by the ozone generator, the photo-catalyst filters and the impregnated carbon, so that hydrogen sulfide $H_2S$, methylmercaptan $CH_3SH$, ethylmercaptan $C_2H_5SH$, methyl sulfide $H_3SCH_3$, and methyl disulfide $CH_3SSCH_3$ can be deodorized with high efficiency.

Furthermore, according to the present invention, additional remarkable effects, such as mentioned below, will be achieved more than mere the deodorization effect of each of ozone generator, the photo-catalyst filter and the impregnated carbon.

That is, in the case of the impregnated carbon which is formed by impregnating an activated carbon with potassium hydroxide (KOH), since the potassium hydroxide has alkaline, sulfur component in odor, which has acid, will be easily captured. Moreover, since the surface of the activated carbon of the impregnated carbon is present in the ozone atmosphere, the activated carbon will be easily oxidized and deteriorated by the ozone. However, since the potassium hydroxide also exists on the surface of this activated carbon, the oxidization and deterioration of the activated carbon by the ozone will be prevented or effectively reduced by the potassium hydroxide, thereby elongating the usable time of the activated carbon.

Still furthermore, the potassium hydroxide adsorbs the hydrogen sulfide in the contaminated air, and under the hydrogen sulfide adsorbed state, reacts with the ozone to thereby produce a reactant other than the hydrogen sulfide. Although this reactant may include odor component, it is adsorbed by the activated carbon or potassium hydroxide, so that the external releasing of the odor component outside the case body can be prevented or remarkably reduced.

In addition, since the impregnated carbon is disposed on the downstream side of the ozone generator and the photo-catalyst filter, when the contaminated air passes through the impregnated carbon, the odor component has been deodorized twice by the ozone generator and the photo-catalyst filter, so that the odor density has been reduced on the upstream side of the impregnated carbon. For this reason, the quantity of the odor component to be adsorbed by the impregnated carbon can be reduced, so that the use time of the impregnated carbon can be elongated, and the requirement for the impregnated carbon to be exchanged will be reduced, contributing simplification of maintenance.

Still furthermore, since the impregnated carbon having a high deodorization performance adsorbing the odor component is disposed downstream side in the air-flow passage, the odor density of the contaminated air exhausted from the downstream side of the impregnated carbon can be reduced.

**Claims**

1. A deodorization apparatus comprising:

   a case body formed therein with an air-flow passage through which air flows;
   an ozone generator, for generating ozone, disposed in an air-flow passage in the case body;
   a photo-catalyst filter unit disposed in the air-flow passage in the case body and provided with a photo-catalyst filter; and
   an impregnated carbon disposed on a downstream side of at least one of the ozone generator and the photo-catalyst filter and formed by impregnating an activated carbon adsorbing an odor component with at least one component of iron oxide, potassium hydroxide, nickel nitrate.

2. A deodorization apparatus according to claim 1, wherein said ozone generator is a light source radiating a light having wavelengths of 185nm and 254nm.

3. A deodorization apparatus according to claim 1 or 2, wherein said photo-catalyst filter is formed by fixing a photo-catalyst to a glass wool by using fluororesin.

4. A deodorization apparatus according to claim 2, wherein the light sources for generating the ozone are disposed to both side portions of the photo-catalyst filter in the air-flow direction.

5. A deodorization apparatus according to any one of claims 1 to 4, wherein said impregnated carbon is accommodated in a container, through which the air passes, and said impregnated carbon container and said photo-catalyst filter are formed into a unit to be detachable to the case body.

6. A deodorization apparatus according to any one of claims 1 to 5, further comprising a support member for detachably supporting the impregnated carbon unit and the photo-catalyst filter in the case body and an elastic member for elastically supporting the impregnated carbon unit and the photo-catalyst filter in said support member.

7. A deodorization apparatus according to any one of claims 1 to 6, wherein said photo-catalyst filter has a bent surface whose air-flow surface is bent.

8. A deodorization apparatus according to any one of claims 1 to 7, wherein an exhaust means for externally exhausting air in the case body is disposed downstream side of the impregnated carbon.

9.  A deodorization apparatus according to claim 1, further comprises a safety device disposed in the case body for blocking or stopping an operation of the ozone generator at a time of detecting that the ozone generated by the ozone generator leaks outside the case body.

10. A deodorization apparatus according to claim 1, wherein when an air-flow rate in the case body is 10 to 18 $m^3$/hr, an impregnated carbon having a grain diameter of 4 to 6 mm fills a unit case having a thickness of 70 to 130 mm in the air-flow direction, said unit case being detachably mounted to the case body.

11. A deodorization apparatus according to claim 10, wherein said impregnated carbon unit case has a thickness adjustable in the air-flow direction.

12. A deodorization apparatus according to claim 9, wherein said case body is provided with an open/close lid for the case body and said safety device comprises a first detection means for blocking or stopping the operation of the ozone generator upon detection of an opened state of said open/close lid.

13. A deodorization apparatus according to claim 9, wherein said safety device comprises a second detection means for blocking or stopping the operation of the ozone generator upon detection of a state that the impregnated carbon unit is mounted to a portion other than a predetermined position in the case body.

14. A deodorization apparatus according to claim 12 or 13, wherein said first and second detection means are disposed downstream side of the impregnated carbon in the air-flow direction.

FIG. 1

EP 1 417 978 A1

1   DEODORIZATION APPARATUS
2   CASE BODY
2a  CASE BODY PORTION
2c  OPENED UPPER END OF CASE BODY PORTION
2d  UPPER LID OF CASE BODY PORTION
2d  PACKING OF UPPER LID
2f, 2g SIDE WALL
2h  INNER BOTTOM SURFACE
2i  AIR-FLOW PASSAGE
3   INTAKE PIPE
5   EXHAUST FAN
6   WATER REMOVAL AIR-FILTER

7   FIRST PHOTO-CATALYST FILTER
8   FIRST LAMP UNIT
9   SECOND PHOTO-CATALYST FILTER
10  SECOND LAMP UNIT
11  THIRD PHOTO-CATALYST FILTER
12  ADSORBENT UNIT
13  ULTRAVIOLET LAMP
15  ELECTRICAL PART BOX
16a, 16b A PAIR OF GUIDE RAILS
17  LERF SPRING

# FIG. 2

13a MOUTH PIECE
14 SOCKET

4 PATCHING LATCH
4a HOOK
4b LATCH
4c LEVER
4d BASE
4e PATCHING LATCH BODY

# FIG. 3

FIG. 4

FIG. 5

FIG. 6

101

EP 1 417 978 A1

| | |
|---|---|
| 101 | DEODORIZATION APPARATUS |
| 102 | CASE BODY |
| 102a | CASE BODY PORTION |
| 102b | OPENED UPPER END OF CASE BODY PORTION |
| 102c | UPPER LID OF CASE BODY PORTION |
| 102d | PACKING OF UPPER LID |
| 102f, 102g | SIDE WALL |
| 102h | INNER BOTTOM SURFACE |
| 102i | AIR-FLOW PASSAGE |
| 103 | INTAKE PIPE |
| 105 | EXHAUST FAN |
| 106 | WATER REMOVAL AIR-FILTER |
| 107 | FIRST PHOTO-CATALYST FILTER |
| 108 | LAMP UNIT |
| 109 | SECOND PHOTO-CATALYST FILTER |
| 110 | IMPREGNATED CARBON UNIT |
| 111 | ULTRAVIOLET LAMP |
| 113 | ELECTRICAL PART BOX |
| 114a, 114b | A PAIR OF GUIDE RAILS |
| 115 | LERF SPRING |
| 116 | UPPER LID DETECTION LIMIT SWITCH |
| 117 | IMPREGNATED CARBON DETECTION LIMIT SWITCH |

FIG. 7

102c  UPPER LID
110d, 110e  THROUGH BOLT
110f  NUT
111a  MOUTH PIECE
112  SOCKET
118  UPPER LID ALARM LAMP
119  IMPREGNATED CARBON ALARM LAMP

```
104  PATCHING LATCH
104a HOOK
104b LATCH
104c LEVER
104d BASE
104e PATCHING LATCH BODY
```

# FIG. 8

| IMPREGNATED CARBON UNIT THICKNESS [mm] | AIR-FLOW VELOCITY [m/s] | AIR-FLOW RATE [m³/hr] |
|---|---|---|
| 50 | 3. 4 | 24. 2 |
| 60 | 3. 1 | 21. 8 |
| 70 | 2. 8 | 19. 6 |
| 80 | 2. 5 | 17. 7 |
| 90 | 2. 2 | 15. 9 |
| 100 | 2. 0 | 14. 3 |
| 110 | 1. 8 | 12. 9 |
| 120 | 1. 6 | 11. 6 |
| 130 | 1. 5 | 10. 4 |
| 140 | 1. 3 | 9. 4 |

# FIG. 9

FIG. 10

102d  102c

116b

116

116d

116a

116c

102k

102j

# FIG. 11

110

102g

117a

117b

117c

121

# FIG. 12

# EP 1 417 978 A1

## INTERNATIONAL SEARCH REPORT

| International application No. |
|---|
| PCT/JP01/11558 |

**A. CLASSIFICATION OF SUBJECT MATTER**
Int.Cl$^7$ A61L9/015, 9/00, 9/014

According to International Patent Classification (IPC) or to both national classification and IPC

**B. FIELDS SEARCHED**

Minimum documentation searched (classification system followed by classification symbols)
Int.Cl$^7$ A61L9/015, 9/00, 9/014

Documentation searched other than minimum documentation to the extent that such documents are included in the fields searched

| | | | |
|---|---|---|---|
| Jitsuyo Shinan Koho | 1926–1996 | Jitsuyo Shinan Toroku Koho | 1996–2002 |
| Kokai Jitsuyo Shinan Koho | 1971–2002 | Toroku Jitsuyo Shinan Koho | 1994–2002 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP, 9-206558, A (Shinnikka Kosa K.K.), 12 August, 1997 (12.08.97), Full text (Family: none) | 1–14 |
| Y | JP, 52-63882, A (Mitsubishi Heavy Industries, Ltd.), 26 May, 1977 (26.05.77), Full text (Family: none) | 1–14 |
| Y | JP, 62-142559, A (Zenko Kagaku Kenkyusho K.K.), 25 June, 1987 (25.06.87), Full text (Family: none) | 1–14 |
| Y | JP, 8-994, A (Kuraray Chemical K.K.), 09 January, 1996 (09.01.96), Full text (Family: none) | 1–14 |

| ☒ Further documents are listed in the continuation of Box C. | ☐ See patent family annex. |
|---|---|

\* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 April, 2002 (01.04.02) | 16 April, 2002 (16.04.02) |

| Name and mailing address of the ISA/ | Authorized officer |
|---|---|
| Japanese Patent Office | |
| Facsimile No. | Telephone No. |

Form PCT/ISA/210 (second sheet) (July 1998)

27

## INTERNATIONAL SEARCH REPORT

| International application No. |
|---|
| PCT/JP01/11558 |

C (Continuation).    DOCUMENTS CONSIDERED TO BE RELEVANT

| Category* | Citation of document, with indication, where appropriate, of the relevant passages | Relevant to claim No. |
|---|---|---|
| Y | EP, 633064, A1  (Ishihara Sangyo Kaisha Ltd.),<br>11 January, 1995 (11.01.95),<br>Full text<br>& AU 9464810 A          & CA 2126418 A<br>& US 5547823 A          & TW 279175 A<br>& CN 1101591 A          & JP 11-124546 A | 3 |
| Y | JP, 10-118169, A  (Isuzu Motors Ltd.),<br>12 May, 1998 (12.05.98),<br>Par. No. [0010]<br>(Family: none) | 9-14 |
| Y | JP, 10-253105, A  (Maruzen Create Inc.),<br>25 September, 1998 (25.09.98),<br>Par. No. [0022]<br>(Family: none) | 9-14 |

Form PCT/ISA/210 (continuation of second sheet) (July 1998)